# EUROPEAN PATENT SPECIFICATION

(11) **EP 4 434 493 B1**
(45) Date of publication and mention of the grant of the patent: **28.10.2026**
(21) Application number: 22894908.7
(22) Date of filing: 17.11.2022
(51) Int. Cl.: A61F 2/24, A61F 2/966, A61F 2/02

(54) **SPLIT-TYPE INTERVENTIONAL TRICUSPID VALVE SYSTEM CAPABLE OF ACCURATELY ANCHORING**
GETEILTES INTERVENTIONELLES TRIKUSPIDALKLAPPENSYSTEM MIT FÄHIGKEIT ZUR GENAUEN VERANKERUNG
SYSTÈME DE VALVULE TRICUSPIDE D'INTERVENTION DE TYPE FENDU CAPABLE D'ANCRAGE AVEC PRÉCISION

(30) Priority: 17.11.2021 CN 202111361301
(43) Date of publication of application: 25.09.2024
(73) Proprietor: Beijing Balance Medical Technology Co., Ltd., Beijing 102200 (CN)
(72) Inventor: JIN, Lei, Beijing 102200 (CN); HUANG, Huanlei, Guangzhou, Guangdong 510080 (CN); WANG, Zhenzhong, Guangzhou, Guangdong 510080 (CN); LI, Liyan, Beijing 102200 (CN); FAN, Zhihao, Beijing 102200 (CN); MU, Hong, Beijing 102200 (CN); WU, Jia, Beijing 102200 (CN); WU, Kangjian, Beijing 102200 (CN)
(74) Representative: Braunpat AG
(86) International application number: PCT/CN2022/132606
(87) International publication number: WO 2023/088377

(56) References cited:
- WO-A1-2015/077599
- WO-A2-2013/114214
- CN-A- 104 220 027
- CN-A- 113 473 947
- US-A1- 2008 319 526
- US-A1- 2010 217 382
- US-A1- 2014 243 964
- US-A1- 2016 302 918
- US-A1- 2018 338 832
- US-A1- 2019 224 005
- US-A1- 2019 321 171
- US-A1- 2021 154 010
- US-A1- 2021 338 419

## Description

### Technical Field

The invention relates to an artificial biological heart valve, in particular to a split type precisely-anchorable transcatheter tricuspid valve system.

### Background Art

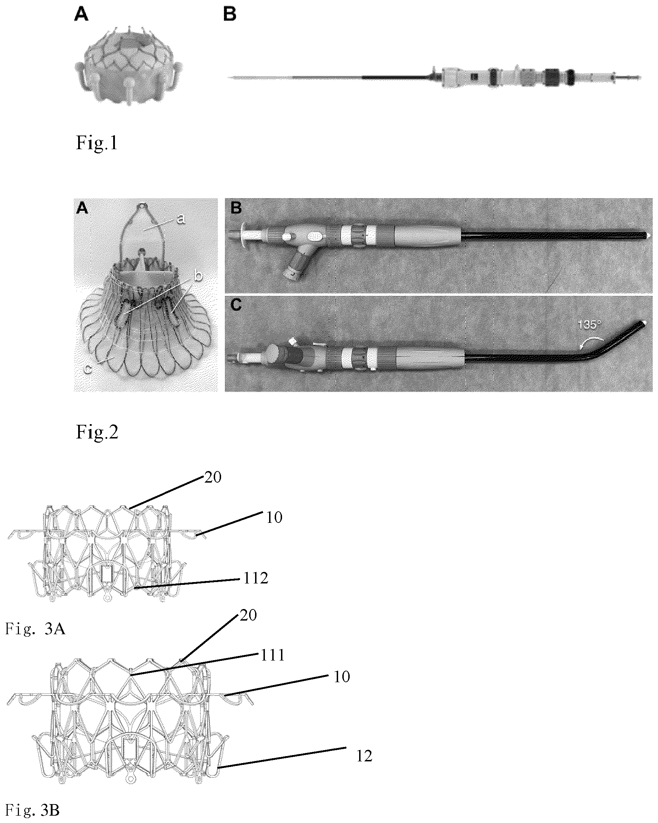

The tricuspid valve serves as a one-way valve for the blood to flow back to the first inlet of the heart, often experiences regurgitation (TR) due to valve dysfunction, mainly resulting in secondary lesions, it is commonly caused by valve lesions in other valve sites, if the condition continues to worsen and cannot be treated in a timely manner, a large amount of reflux can lead to heart failure or even death. Compared with the left mitral valve and aortic valve, the research and treatment of the right tricuspid valve and pulmonary valve have been overlooked in the past, and their harm has been underestimated. In recent years, the number of cases for surgical tricuspid valve repair and replacement increases year by year. For elderly patients with multiple underlying diseases, surgical valve replacement not only carries high risks but also often fails to achieve satisfactory treatment outcomes. Due to the large number of TR patients in China who have to be treated, with the effective treatment of transcatheter aortic valve (TAVR) in elderly or high-risk patients undergoing traditional surgical aortic valve replacement, there has been significant progress in the research of transcatheter tricuspid valve repair and replacement instruments in the past decade. Among them, transcatheter tricuspid replacement is considered an etiology treatment for such patients. However, due to the fact that the tricuspid valve as the intracavitary valve of the right heart, has a larger non planar elliptical and softer valve ring tissue compared to the mitral valve, mainly due to functional regurgitation, most of the valve ring has no calcification, the thinner right ventricular wall compared to the left heart, the right coronary artery adjacent to the valve ring, the more complex perivalvular environment adjacent to the conduction bundle, and the significant deformation of the valve ring and supravalvular and subvalvular with each cardiac cycle, the diversity and complexity of these anatomical forms and structures make it difficult for the transcatheter artificial biological tricuspid valve to be designed with supravalvular and perivalvular radial support, similar to aortic valves. According to the existing transcatheter tricuspid valve products and the patents disclosed in the existing similar products, from the design concept and the structural characteristics, the valve structure (FIGS. 1 -2) is implanted and integrated through a catheter at one time, and it is difficult to meet the personalized complex pathological anatomical environment of the tricuspid valve site.

US patent application No. US 2016/302918 A1 discloses a low-profile prosthetic heart valve for replacing a mitral valve. A prosthetic valve assembly comprises a valve component comprising a radially compressible and expandable frame and a valve structure supported inside of the frame. The valve structure is configured to allow blood to flow through the valve component in one direction and block the flow of blood in the opposite direction. The assembly further comprises a radially compressible and expandable anchor comprising an annular base and a plurality of cantilevered fixation members extending from the base. The fixation members are configured to pivot inwardly toward the valve component when the valve component is radially expanded within the anchor.

US patent application No. US 2019/224005 A1 discloses a docking device for aortic valve replacement. A docking device for aortic valve replacements is a self-expanding nitinol structure including a lattice forming a tubular (e.g., cylindrical) upper framework or cage, and three struts forming a lower structure. The struts are formed by elongated loops that are attached to the lattice or that are an integral part of the upper framework. The struts extend down beneath the lattice, eventually forming "feet" to anchor at the base of the aortic cusps. The docking device can be positioned near a sinotubular junction of a patient. An aortic valve replacement is locked inside the docking device.

PCT patent application No. WO 2013/114214 A2 discloses mitral valve docking devices, systems and methods. Various systems, devices and methods associated with the placement of a dock or anchor for a prosthetic mitral valve. The anchor may take the form of a helical anchor having multiple coils and/or a stent-like structure. Various methods include different levels of minimal invasive procedures for delivering the prosthetic valve anchor and prosthetic valve, as well as tissue anchors for plication or other purposes to the mitral valve position in the heart.

US patent application No. US 2021/338419 A1 discloses modular valve replacement systems and associated devices and methods of use. The present technology relates to modular valve replacement systems for treating valve-related cardiac disorders. In select embodiments, the modular valve replacement system includes a fixation device and a permanent valve assembly. The fixation device and the permanent valve assembly are delivered separately, enabling use of smaller delivery systems and facilitating less-invasive implant techniques. The fixation device is implanted first and provides a mounting fixture for the subsequently delivered permanent valve assembly. In some embodiments, the permanent valve assembly is at least partially mechanically isolated from the fixation device after the permanent valve assembly is attached to the fixation device. In some embodiments, the fixation device includes a temporary valve assembly that prevents regurgitation until the permanent valve assembly is implanted

US patent application No. US 2019/321171 A1 discloses Prosthetic heart valve devices, prosthetic mitral valves and associated systems and methods. Prosthetic heart valve devices for percutaneous replacement of native heart valves and associated systems and method are disclosed herein. A prosthetic heart valve device configured in accordance with a particular embodiment of the present technology can include an anchoring member having an upstream portion configured to engage with tissue on or near the annulus of the native heart valve and to deform in a non-circular shape to conform to the tissue. The device can also include a mechanically isolated valve support coupled to the anchoring member and configured to support a prosthetic valve. The device can further include an atrial extension member extending radially outward from the upstream portion of the anchoring member and which is deformable without substantially deforming the anchoring member. In some embodiments, the upstream portion of the anchoring member and the extension member may be deformed while the valve support remains sufficiently stable.

US patent application No. US 2008/319526 A1 discloses percutaneous valve, system and method. The apparatus includes a valve having a valve frame, a valve leaflet coupled to the valve frame, and a thread passing over the valve frame to hold the valve frame in a partially deployed state, where removal of the thread allows the valve frame to expand toward a deployed state. Use of the thread to hold the valve frame allows for staged deployment of the valve.

### Summary

The invention is directed to a split type precisely-anchorable transcatheter tricuspid valve system as defined in independent claim 1.
Different from the traditional concept of transcatheter tricuspid valve product design, the invention provides a split type precisely-anchorable transcatheter tricuspid valve system. The split type meaning is that the product consists of two independent instruments of n transcatheter tricuspid valve anchoring stent and antranscatheter artificial biological tricuspid valve, the former is firstly delivered to the tricuspid valve site to be released through a catheter, and then the latter is introduced into the tricuspid valve to assist and deform in the tricuspid valve site through the balloon expandable external force, and are integrated together at the lesion site, so that personalized accurate anchoring mainly on the lesion valve tissue is realized.

Different from the structure of the traditional transcatheter tricuspid valve product, the transcatheter tricuspid valve system is composed of two parts, namely a transcatheter tricuspid valve anchoring stent and a transcatheter artificial biological tricuspid valve the anchoring of the valve and the support of the valve leaflet are divided into two different structures, namely the split transcatheter tricuspid valve anchoring stent is responsible for anchoring of the valve, and the transcatheter artificial biological tricuspid valve is delivered into the pre-interventional anchoring valve frame and is combined with the stent to realize accurate anchoring.

The transcatheter tricuspid valve system of the invention comprises a split transcatheter tricuspid valve anchoring stent and a split type precisely-anchorable artificial biotricuspid valve prosthesis. One of the main contents of the present invention is that the shape and structure of the transcatheter tricuspid anchoring stent has two different anchoring states, namely a first anchoring state after being released by a catheter, and a second anchoring state after being combined with a transcatheter artificial biological tricuspid valve.

The first anchoring state is customized according to the real structure and shape of the tricuspid valve after the personalized image data of the patient is subjected to three-dimensional reconstruction and shape customization design and in-vitro three-dimensional forming processing, so that the return shape after being released through the catheter can be matched with the precise matching and alignment of the supravalvular and subvalvular tissue of the tricuspid valve site of the patient, the atrial surface of the transcatheter tricuspid valve anchoring stent is funnel-shaped to the ventricular surface, and the released form can accurately align with the supravalvular and subvalvular tissue of the tricuspid valve of the patient's dynamic lesion, and forming mutual clamping with it. The processing and shaping of the first anchoring state of the transcatheter tricuspid valve anchoring stent depends on how to achieve the precise matching degree between the transcatheter tricuspid valve anchoring stent and the real anatomical structure of the tricuspid valve of the patient. The real structure of the three-dimensional reconstruction is a digital image model or a three-dimensional printed simulation entity model; the real structure of the three-dimensional reconstruction is a three-dimensional image of a virtual simulation after digital conversion of CT, ultrasonic and nuclear magnetic integrated images and a corresponding three-dimensional printing simulation entity model.

The first anchoring state of the transcatheter tricuspid valve anchoring stent is designed and processed into an umbrella tubular stent structure according to the real anatomical morphological design of the three-tricuspid valve of the patient with three-dimensional reconstruction, that is, three parts of the atrial surface, the ventricular surface and the anchoring stent connecting part therebetween. The ① atrial surface is an umbrella shape, has an umbrella shape matched with the real form of three-dimensional reconstruction of the image data of the atrium surface of the patient, is a first lattice portion, and can be laid on the upper end of the bottom of the right atrium at the bottom of the right atrium at the bottom of the tricuspid valve; the ② ventricular surface is three positioning hook loops which are precisely preset with the boundary positions of the three leaflet leaves of the anterior leaflet, the septum and the posterior leaflet of the tricuspid valve of the patient, and after release, the anchoring stent is positioned on the atrial surface and personalized corresponding to the shape of the patient's right atrium; the ③ the connection part of the anchoring support is a funnel with a round opening and is a second lattice part. So far, it is shown that the first anchoring state of the transcatheter tricuspid valve anchoring stent refers to a personalized three-dimensional structure released from all the catheters in the catheter.

The second anchoring state of the transcatheter tricuspid valve anchoring stent refers to that in the first anchoring state, the transcatheter artificial biological tricuspid valve is delivered into the transcatheter tricuspid valve anchoring stent in the first state by the catheter, the balloon expandable external force is accompanied by the second deformation of the transcatheter tricuspid valve anchoring stent which occurs with the expansion of the transcatheter artificial biological tricuspid valve, the conical shape (funnel shape) of the original first anchoring state is deformed into the final cylindrical shape and is integrated with the expanded transcatheter artificial biological tricuspid valve, and meanwhile, the ventricular surface structure of the transcatheter tricuspid valve anchoring stent in the second anchoring state is anchored with the chordae tendineae and papillary muscle of the tricuspid valve of the patient in a precise and preset combination..

The first anchoring state of the connecting part of the transcatheter tricuspid valve anchoring stent is a three-dimensional shape-memory state of the real anatomical shape and structure of the patient after delivery and release of the catheter, the shape-memory state of the connecting part from the atrial surface to the ventricular surface has a contraction taper, the taper is 5-45 degrees, the anchoring stent connecting part is deformed and expanded to form a cylindrical second anchoring state from the conical funnel-shaped deformation of the first anchoring state depending on the shape of the lesion leaflet of the patient. In the first anchoring state of the transcatheter tricuspid valve anchoring stent, after the positioning hook loop is released through the catheter, the positioning hook loop is inserted into the junction position of the anterior leaflet, the septum and the posterior leaflet of the tricuspid valve of the patient's lesion matched with the positioning hook loop, so as to realize personalized corresponding placement of the atrial surface of a positioning anchoring stent and the right atrium of the patient. The ventricular surface of the tricuspid valve anchoring stent has a plurality of anchoring hook loops, and the anchoring hook loops extend from the connecting part to the right ventricular surface and then be folded, so as to accurately match the number of morphological contours of the real chordae tendineae and subvalvular tissue structure shape of the three-dimensional reconstruction of the subvalvular image data of the patient's lesion. The number, size, shape and folding angle of the anchoring hook loops are personalized and accurately matched with the real chordae tendineae gap, the size and shape of the tricuspid valve leaflet, and the circumferential spacing of the perivalvular tissue from the ventricular wall in three dimensions of the three-dimensional reconstruction of the subvalvular image data of the tricuspid valve of the patient's lesion.

The second anchoring state of the transcatheter tricuspid valve anchoring stent is mainly based on the three-dimensional reconstruction of the tricuspid valve leaflet shape, leaflet area, and the real anatomical structure of the chordae tendineae and papillary muscle beneath the tricuspid valve leaflet based on the patient's tricuspid valve ultrasound images, the shape, size, and bending angle of the positioning hook loop on the ventricular surface of the transcatheter tricuspid valve anchoring stent are designed and processed, so that when the transcatheter artificial biological tricuspid valve is inserted in a gripping state and pressurized and expanded by a pump, the shape and structure match an equal number of anchoring hook loops, and the positional deformation is achieved to achieve the preset final anchoring state, completing the final and accurate tight integrating with the patient's tricuspid valve site and subvalvular tissue.

The transcatheter tricuspid valve anchoring stent is characterized in that the transcatheter tricuspid valve anchoring stent is placed in a compressed state in a catheter, and is presented as a first anchoring state after being released through a catheter, and then is transformed into a second anchoring state in combination with a transcatheter artificial biological tricuspid valve, is 3 positioning hook loops of the anchoring stent connecting part, and is accurately inserted into the three interfaces of the anterior leaflet, the septum and the posterior leaflet of the tricuspid valve after being released through a catheter, so that the whole stent is positioned, the positioning hook loop not only positions the shape of the atrial surface of the transcatheter tricuspid valve anchoring stent and the shape of the right atrium of the patient and the amplitude of the systolic relaxation of the right atrium of the cardiac cycle, but also positions several anchoring hook loops of the ventricular surface of the transcatheter tricuspid valve anchoring stent in the chordae tendineae gaps under the leaflet of the tricuspid valve of the patient's lesion corresponding to the insertion, clamping, and transition of the transcatheter tricuspid valve anchoring stent to a second anchoring state, and these anchoring hook loops can be tightly intertwined and pre-set with the subvalvular tissue.

The transcatheter tricuspid valve anchoring stent is subjected to a first anchoring state after being released by a catheter and then is deformed into a second anchoring state in combination with a transcatheter artificial biological tricuspid valve, the atrial surface end portion of the connecting part of the transcatheter tricuspid valve anchoring stent is provided with a plurality of fixed support rods for embedding the transcatheter artificial biological tricuspid valve stent, and the fixed support rods are bent along the axial direction of the atrial surface and the ends thereof bend toward the axis of the anchoring stent; the connecting part of the tricuspid valve anchoring stent is provided with a plurality of end centripetal bending hooks for embedding the outflow end of the transcatheter artificial biological tricuspid valve stent, and the atrial surface end portions of the connecting parts of the centripetal bending hooks and the tricuspid valve anchoring stent are provided with a plurality of fixed support rods used for embedding the atrial end of the transcatheter artificial biological tricuspid valve stent in the upper and lower coaptation, and the ventricular side can be prevented from being displaced when the transcatheter artificial biological tricuspid valve is released. In the first anchoring state of the transcatheter tricuspid valve anchoring stent, the fixed support rod maintains an angle consistent with the anchoring stent connecting part, and in the second anchoring state of the tricuspid valve anchoring stent, the plurality of fixed support rods are axially parallel to the coaptation circumference, so that the end of the fixed support rod is embedded on the stent of the inflow end of the transcatheter artificial biological tricuspid valve, and structurally integrate with the transcatheter artificial biological tricuspid valve to realize zero displacement of the release of the transcatheter artificial biological tricuspid valve. The fixed support rod is 3-12, preferably 9. The first lattice portion and the second lattice portion of the transcatheter tricuspid valve anchoring stent are formed of a unit lattice composed of a compressible diamond lattice, a V-shaped lattice and / or a hexagonal or polygonal lattice, and the first lattice portion is adaptively connected to the second lattice portion. An outer periphery of the lattice portion of the atrial surface of the transcatheter tricuspid valve anchoring stent is spaced apart from the atrial wall of the patient by 1-2 mm, preferably by 1.5 mm. An inner peripheral edge diameter of the second lattice portion of the transcatheter tricuspid valve anchoring stent is matched with an outer diameter of various corresponding size specifications of the transcatheter artificial biological tricuspid valve. A surface of the transcatheter tricuspid valve anchoring stent is partially or completely coated with a layer of medical polymer film. The atrial and ventricular surfaces of the tricuspid valve anchoring stent and the connecting parts of the anchoring stent are three-dimensional shaped structures formed by laser integrated cutting or the atrial and ventricular surfaces and the connecting parts of the anchoring stent are reconnected after a split processing. The anchoring stent is a metallic material or a non-metallic material having shape-memory reshape properties. The anchoring stent is made of a nickel-titanium alloy material.

Further, the transcatheter artificial biological tricuspid valve comprises a radially compressible cobalt-chromium alloy stent which can be expanded after balloon expansion, or a radially compressible self-expanding nickel-titanium alloy stent, and three fan-shaped leaflets arranged on the inner side of the stent, any of the fan-shaped leaflets has a free edge, an arc-shaped bottom edge and leaflet boundary connecting parts extending on the two sides, and the stent is a metal net tube or a valve stent which can support three commissures of three leaflet interfaces and can be crimped. The valve stent is a cobalt-based alloy cobalt or chromium alloy or a nickel-titanium alloy. The transcatheter artificial biological tricuspid valve delivery kit comprises a transcatheter artificial biological tricuspid valve delivery device, a guide sheath, a valve holder, and a charging pump. The transcatheter tricuspid valve anchoring stent delivery device and the transcatheter artificial tricuspid valve delivery device can be delivered from the inferior vena cava via the femoral vein, or from the superior vena cava via the jugular vein or subclavian vein to the tricuspid valve site. The transcatheter tricuspid valve anchoring stent is firstly guided into the diseased tricuspid valve site of the patient through the catheter and released into the first anchoring state, and then the transcatheter artificial biological tricuspid valve is sent into the anchoring stent through the catheter, and as the valve is expanded, the transcatheter tricuspid valve anchoring stent is expanded to the second anchoring state, and meanwhile, the stent connecting part and the transcatheter artificial biological tricuspid valve are fitted and the ventricular surface of the stent completes further tight combination with the subvalvular structure to form final anchoring.

In this invention, each completion of a personalized preset transcatheter artificial biological tricuspid valve treatment process for a specific patient, all of the related data is used as an independent data unit, a large amount of personalized data is accumulated, and intelligent, large-scale and industrialization of the split type precise anchoring intervention tricuspid system is achieved through artificial intelligence.

### Brief Description of Drawings

FIG. 1 shows a physical image of an EVOQUE transcatheter artificial biological tricuspid valve.
FIG. 2 shows physical image of Lux-Valve transcatheter artificial biological tricuspid valve.
FIGS. 3A-C show a schematic diagram of a combination of a split type anchoring stent and a transcatheter artificial biological tricuspid valve with different supravalvular and subvalvular structures according to an embodiment of the present invention.
FIG. 4A -4C show a schematic diagram of a split type anchoring stent with different supravalvular and subvalvular structures according to an embodiment of the present disclosure.
FIG. 5 shows a schematic diagram of an atrial surface of a split type anchoring stent according to an embodiment of the present disclosure.
FIG. 6 shows a schematic diagram of a ventricular surface and a stent connecting part of a split type anchoring stent according to an embodiment of the present disclosure.
FIGS. 7A-C show a schematic diagram of fixed support rods and centripetal bending of a split type anchoring stent according to an embodiment of the present disclosure.
FIG. 8A -8B show a schematic diagram of a first anchoring state after a transcatheter tricuspid valve anchoring stent is implanted into a human body according to an embodiment of the present disclosure.
FIGS. 9A-B show a schematic diagram of a second anchoring state after a transcatheter tricuspid anchoring stent is implanted into a human body according to an embodiment of the present disclosure.
FIG. 10 shows a schematic diagram of anchoring hook loops and chordae tendineae secondary anchoring after a transcatheter tricuspid anchoring stent is implanted into a human body according to an embodiment of the present disclosure.
FIG. 11A -11B show a schematic diagram of a transcatheter artificial biological tricuspid valve before and after compression according to an embodiment of the present disclosure.
FIG. 12 shows a schematic diagram of a delivery system according to an embodiment of the present disclosure.
FIG. 13 shows a schematic diagram of a loading of a transcatheter tricuspid valve anchoring stent through atrial septum by a transfemoral approach according to an embodiment of the present invention.
FIG. 14A-D show a schematic diagram of a process of a transcatheter tricuspid valve anchoring stent through atrial septum by a transfemoral approach according to an embodiment of the present invention.
FIG. 15A-B show a schematic diagram of a process of feeding a transcatheter artificial biological tricuspid valve through atrial septum into an anchoring stent by a transfemoral approach according to an embodiment of the present invention.
FIGS. 16A-C show a schematic diagram of a process of introducing a composite approach into a transcatheter tricuspid valve anchoring stent according to an embodiment of the present invention.
FIGS. 17A-D show a schematic views of a process of feeding a transcatheter artificial biological tricuspid valve into an anchoring stent via a composite approach according to an embodiment of the present invention.

### Detailed Description

By combining the accompanying drawings and the specific description of the present invention mentioned above, it is possible to have a clearer understanding of the details of the present invention. However, the specific embodiments of the present invention described herein are only for the purpose of explaining the present invention and cannot be understood in any way as a limitation of the present invention. Under the guidance of the present invention, technicians may conceive any possible variations based on the present invention, which should be considered within the scope of the present invention.

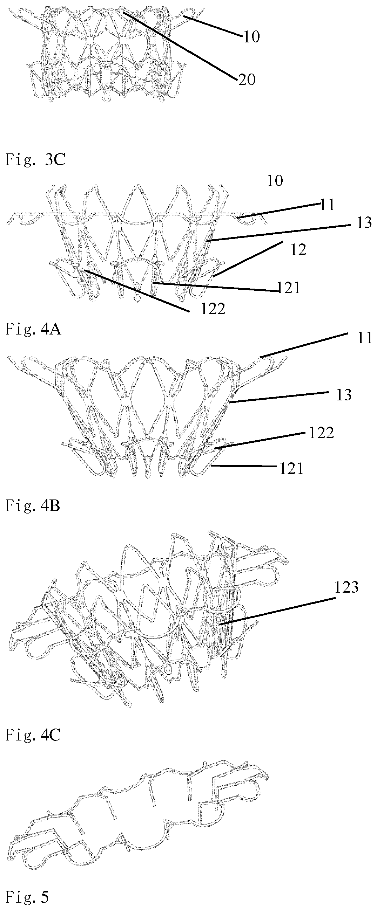

According to the present invention, the split type precisely-anchorable transcatheter tricuspid valve system comprises a split transcatheter tricuspid valve anchoring stent 10 and a transcatheter artificial biological tricuspid valve 20, the shape and structure of the transcatheter tricuspid valve anchoring stent are matched with the anatomical structure of the tricuspid valve real lesion after the patient's image data is subjected to three-dimensional reconstruction, the transcatheter tricuspid valve anchoring stent is firstly delivered to the tricuspid valve site release and deformation of the patient lesion through a catheter, and is in alignment engagement with the supravalvular 40 and subvalvular 50 tissue of the tricuspid valve site of the patient; the transcatheter artificial biological tricuspid valve is delivered into the transcatheter tricuspid valve anchoring stent which has been aligned and engaged with the tissue through a catheter, the transcatheter artificial biological tricuspid valve is released and deformed and expanded to a functional state, the transcatheter tricuspid valve anchoring stent is deformed again to be combined with the expanded transcatheter artificial biological tricuspid valve, and meanwhile, the re-deformation of the transcatheter tricuspid valve anchoring stent enables the rebinding of the anchoring stent and the tricuspid valve and subvalvular tissue of the lesion to achieve final anchoring of the transcatheter artificial biological tricuspid valve. Since the real lesion of each patient is not the same, the transcatheter tricuspid valve anchoring stent designed according to the image data of the patient through three-dimensional reconstruction is not the same, it will be adjusted according to the real situation of the patient, as shown in FIGS. 3A-C, it is a combined schematic diagram of the split type anchoring stent and the transcatheter artificial biological tricuspid valve with three types of the different supravalvular and subvalvular structures, but the general structure and constitution are based on the same design ideas and concepts.

Referring to FIGS. 4-6, a transcatheter tricuspid valve anchoring stent 10 according to the present application, namely a funnel-shaped stent structure shaped as an atrial surface large ventricular surface, is an umbrella tubular stent structure, comprising an atrial surface 11, a ventricular surface 12 and an anchoring stent connecting part 13 therebetween, wherein the atrial surface is an umbrella sheet with an umbrella shape matched with the real shape of the three-dimensional reconstruction of the atrial surface image data of the patient, that is, the first lattice portion; the ventricular surface 12 is an anchoring hook loop 122 which is precisely preset with the three-leaflet boundary position of the anterior leaflet, the septum and the posterior leaflet of the tricuspid valve of the patient, and a plurality of anchoring hook loops 122 matched with the real shape of the three-dimensional reconstruction of the image data of the tricuspid valve of the patient; the anchoring stent connecting part 13 is a small circular opening funnel-shaped structure from the atrial surface to the ventricular surface, the length of the connecting part matches the height of the corresponding transcatheter artificial biological tricuspid valve, and has a second lattice portion which can be expanded into a cylindrical shape. As shown in FIGS. 4A -5C, the schematic diagram of the split type anchoring stent with the different supravalvular and subvalvular structures is consistent, but due to matching lesion tissues of different patients, the atrial surface 11 of different degrees of curvature, the positioning hook loop 121, and the number, angle, length and the like of the anchoring hook loop 122 are designed. In other words, the shape of the atrial surface of the transcatheter tricuspid valve anchoring stent, the size of the covering area, the shape, the number, the length and the angle and the structural relationship of the ventricular surface of the stent and the anchoring hook loop are all according to the pre-operative CT image data of the individual of the patient, after three-dimensional reconstruction (3mensio), the real structures of the atrium (supravalvular) and ventricle (subvalvular) of the patient, as well as the corresponding matching of the various diameter limit structures measured by reference to three-dimensional ultrasound images with the real dimensions, were designed, and based on this, the processing drawings of the interventional tricuspid valve anchoring stent were designed, a personalized tricuspid valve anchoring stent was finally customized through the three-dimensional laser cutting and three-dimensional forming processing of specific nickel titanium memory alloy pipes.

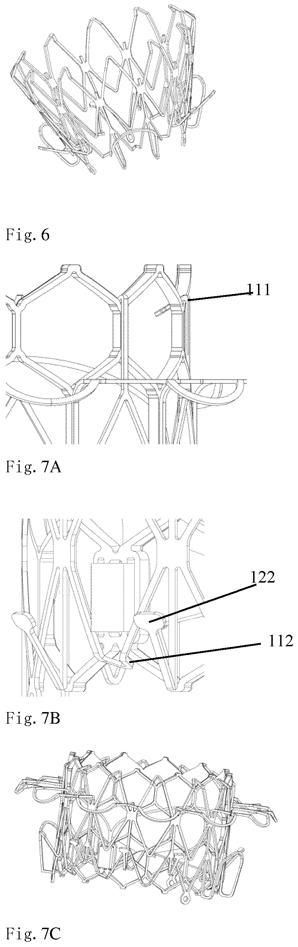

Referring to FIGS. 7A -7C, in order to make the combination of the anchoring stent and the transcatheter artificial biological tricuspid valve more firm, the end portion of the atrial surface 11 of the connecting part of the transcatheter tricuspid valve anchoring stent is provided with a plurality of fixed support rods 111 for embedding the transcatheter artificial biological tricuspid valve stent, and the end portion of the fixed support rod extending axially along the atrial surface direction is bent towards the axis of the anchoring stent. Alternatively, the connecting part of the tricuspid valve anchoring stent is provided with a plurality of end centripetal bending hooks 112 for embedding the outflow end of the transcatheter artificial biological tricuspid valve stent, and similar centripetal bending hooks can also replace the fixed support rod on the atrial surface. In the first anchoring state of the transcatheter tricuspid valve anchoring stent, the fixed support rod 111 maintains an angle consistent with the anchoring stent connecting part, and in the second anchoring state of the tricuspid valve anchoring stent, the plurality of fixed support rods 111 are axially parallel to the coaptation circumference, so that the end of the fixed support rod is embedded on the stent of the inflow end of the transcatheter artificial biological tricuspid valve, and the transcatheter artificial biological tricuspid valve is fixed to prevent displacement to the atrial surface. The fixed support rod 111 is 3-12.

The first lattice portion and the second lattice portion of the transcatheter tricuspid valve anchoring stent are formed of a unit lattice composed of a compressible diamond lattice, a V-shaped lattice and / or a hexagonal or polygonal lattice, and the first lattice portion is adaptively connected to the second lattice portion. An outer periphery of the lattice portion of the atrial surface of the transcatheter tricuspid valve anchoring stent is spaced apart from the atrial wall of the patient by 1-2 mm, preferably by 1.5 mm. An inner peripheral edge diameter of the second lattice portion of the transcatheter tricuspid valve anchoring stent is matched with an outer diameter of various corresponding size specifications of the transcatheter artificial biological tricuspid valve. A surface of the transcatheter tricuspid valve anchoring stent is partially or completely coated with a layer of medical polymer film. The atrial and ventricular surfaces of the tricuspid valve anchoring stent and the connecting parts of the anchoring stent are three-dimensional shaped structures formed by laser integrated cutting or the atrial and ventricular surfaces and the connecting parts of the anchoring stent are reconnected after a split processing. The anchoring stent is a metallic material or a non-metallic material having shape-memory reshape properties, for example, a nickel-titanium alloy material.

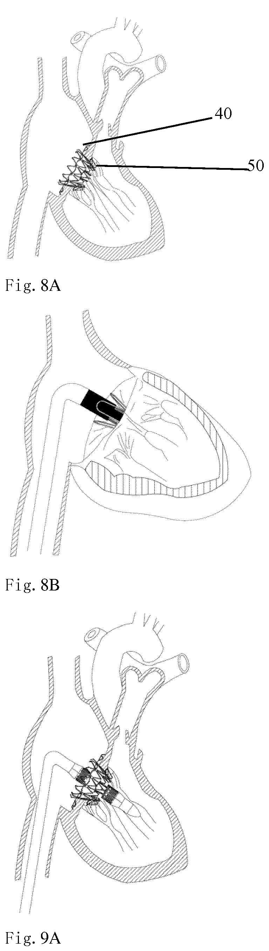

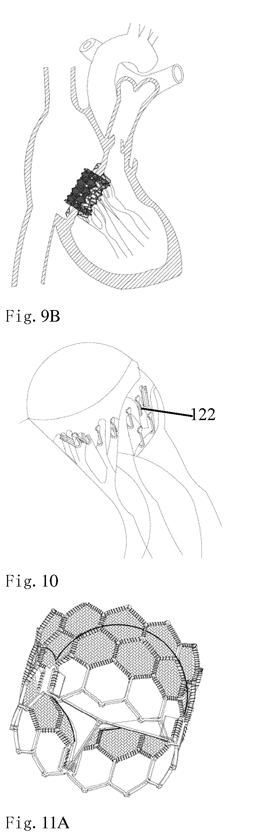

The above process involves manufacturing a tricuspid valve anchoring stent based on real patient imaging data as the pre grip state of the stent, which is also the first anchoring state after the stent is delivered through a catheter to the tricuspid valve and released at the site of the lesion, as shown in FIGS. 8A-8B. The second anchoring state of the transcatheter tricuspid valve anchoring stent is that when the transcatheter artificial biological tricuspid valve is delivered into the anchoring stent through the catheter, by balloon assisted expansion, the valve is expanded (or the nickel-titanium memory alloy valve stent self-expands) to deform the tricuspid valve anchoring stent from the first anchoring state to the second anchoring state, and the deformation force of the stent is combined with the balloon expandable force released by the transcatheter artificial biological tricuspid valve as a whole as shown in FIGS. 9A-9B. And meanwhile, the anchoring stent ventricular surface anchoring hook loop of the chordae tendineae under the alignment insertion valve and the subvalvular tissue, under the action of the transcatheter artificial biological tricuspid valve balloon expandable external force, as the anchoring stent is deformed from the first anchoring state to the second anchoring state, the ventricular surface anchoring hook loop is further tightly combined with the chordae tendineae and the subvalvular tissue 50 to achieve final anchoring, as shown in FIG. 10. At the same time, in the first anchoring state of the anchoring stent, the atrial end fixed support rod or stent bending of the connecting structure of the anchoring stent is deformed into a second anchoring state, and the anchoring stent is axially parallel to the encircling circumference, so that the fixed support rod end or stent bending and the connecting ventricular end of the stent hook back force buckle onto the support rod inserted at both ends of the tricuspid valve stent, and this anchoring stent and the automatic interlocking structure at both ends of the transcatheter artificial biological tricuspid valve stent precisely combine the valve and the anchoring stent to ensure zero displacement of the transcatheter artificial biological tricuspid valve, as shown in FIGS. 7A-7B.

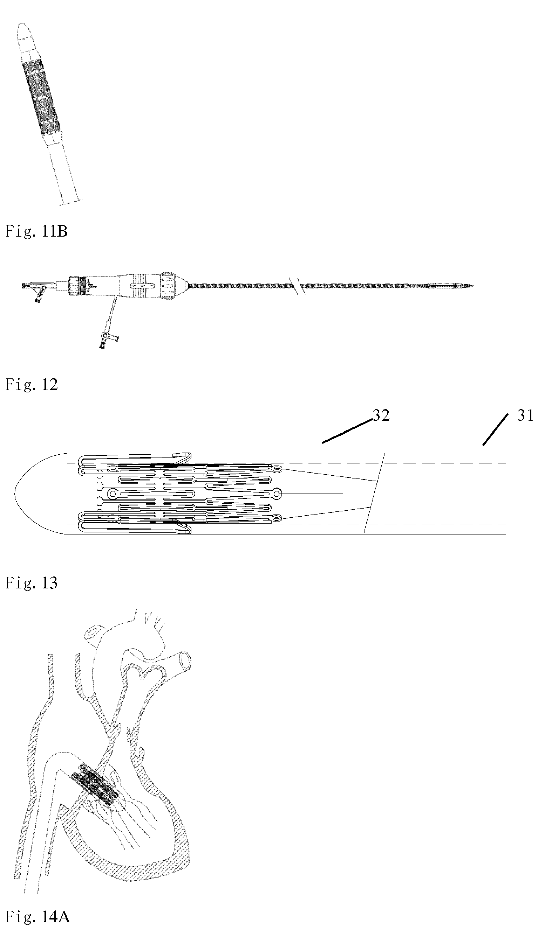

The core point of the invention is as follows: ① the split anchoring stent is designed and the transcatheter artificial biological tricuspid valve is delivered through catheters before and after intervention, and combined in the heart; ② according to the image data of the preoperative lesion tricuspid valve flap and the lower leaflet structure of the patient, the anchoring stent structure is designed in a personalized way and is shaped in three dimensions based on the image data of the supravalvular and subvalvular structure of the tricuspid valve of the patient's preoperative lesion; **③** by utilizing the valve leaflet junction and using a specially designed positioning hook loop, accurately locate the atrial surface shape similarity of the anchoring stent, and align the subvalvular hook loop structure with the chordae tendineae and papillary muscle of the tricuspid valve leaflet; ④ after the release of the anchoring stent, the first anchoring state (funnel-shaped) is determined based on the patient's real pathological anatomical structure, determining the principle and location of the final anchoring, that is, the preset transition state of the second anchoring state (cylindrical), aligning with the personalized pathological anatomical structure, and then, with the help of the deformation force released by the balloon expansion of the tricuspid valve, the anchoring stent is deformed into the second anchoring state of the anchoring stent, so that the transcatheter artificial biological tricuspid valve and the anchoring stent are integrated into a second anchoring state in the heart, and then the clamping is achieved under the supravalvular valve, so that the subvalvular tissue is twisted again between the anchoring stent and the ventricular wall, completing the final anchoring; ⑤ the anchoring stent is deformed from the first state to the second state, the deformation process achieves automatic binding with the transcatheter artificial biological tricuspid valve, and the release manipulation of the transcatheter artificial biological tricuspid valve can be automatically and accurately achieved.
the transcatheter artificial biological tricuspid valve disclosed by the invention, due to the combination of anchoring stent, only serves the reasonable support of the tricuspid valve, the transcatheter artificial biological tricuspid valve comprises a radially compressible cobalt-chromium alloy stent which can be expanded after balloon expansion, or a radially compressible self-expanding nickel-titanium alloy stent, and three fan-shaped leaflets arranged on the inner side of the stent, any of the fan-shaped leaflets has a free edge, an arc-shaped bottom edge and leaflet boundary connecting parts extending on the two sides, and the stent is a metal net tube or a valve stent which can support three commissures of three leaflet interfaces and can be crimped. The valve frame is a cobalt-based alloy cobalt or chromium alloy or a nickel-titanium alloy. Please refer to FIGS. 11A-11B.

The tricuspid valve system of the present application further includes a delivery assembly 30, the delivery assembly comprises a transcatheter tricuspid valve anchoring stent delivery set 31 and a transcatheter artificial biological tricuspid valve delivery set 32. The transcatheter artificial biological tricuspid valve delivery kit comprises a transcatheter artificial biological tricuspid valve delivery device, a guide sheath, a valve holder, and a charging pump. The transcatheter tricuspid anchoring stent delivery device and the transcatheter artificial biological tricuspid valve delivery device are accessible via femoral vein puncture, apical puncture, or left atrial puncture. These techniques are similar to the prior art.

When using the transcatheter tricuspid valve system in this application for interventional treatment, it is necessary to enter through the inferior vena cava via the femoral vein or the superior vena cava via the jugular vein.

FIGS. 13-5 show the transfemoral approach from inferior vena cava.

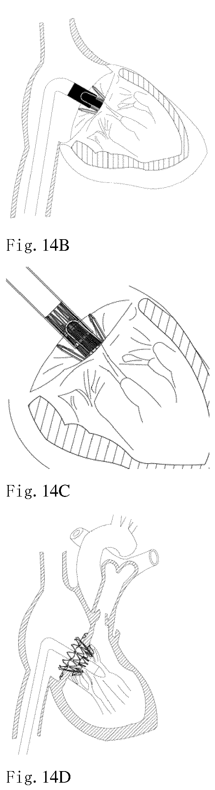

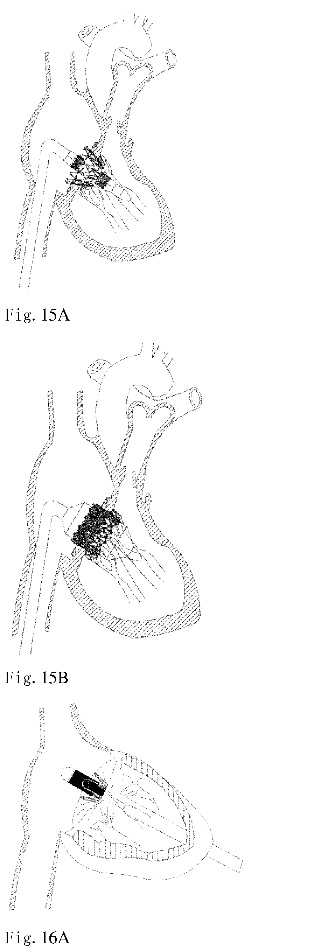

The transfemoral approach from inferior vena cava is the most commonly used and convenient approach. The loaded anchoring stent is delivered into the tricuspid valve of the lesion of the right atrium patient through the transfemoral approach from the inferior vena cava (FIG. 14A), the positioning hook loop is released to complete positioning (FIG. 14B), the anchoring stent is sequentially released on the ventricular surface (FIG. 14C), and the ventricular surface anchoring hook loop is aligned and combined, that is, the anchoring stent is in the first anchoring state (FIG. 14D); the anchoring stent delivery device is withdrawn, the loaded transcatheter artificial biological tricuspid valve is delivered into the anchoring stent along the original path (FIG. 15A), then the transcatheter artificial biological tricuspid valve is assisted by balloon assistance, the anchoring stent is deformed into the second anchoring state, the anchoring stent is automatically and accurately combined with the transcatheter artificial biological tricuspid valve, and meanwhile, the anchoring stent is clamped with the inferior tissue to complete final anchoring (FIG. 15B).

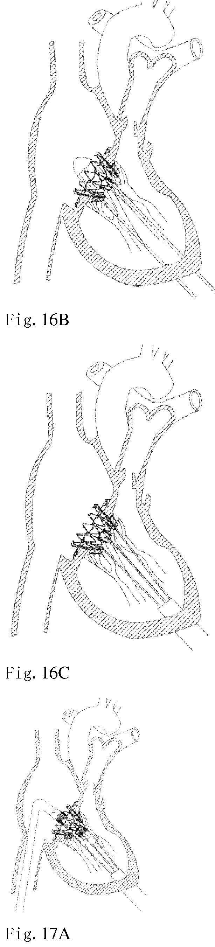

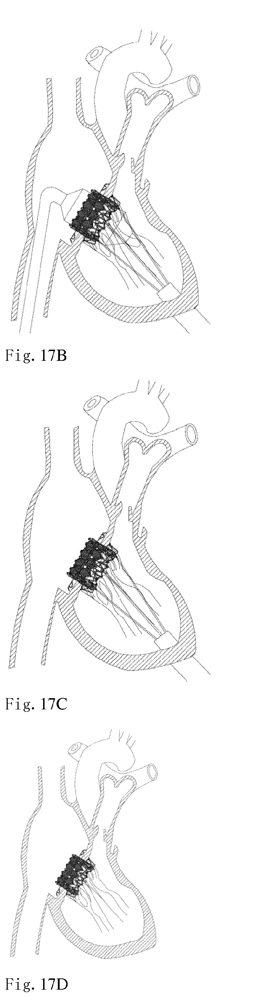

The transcatheter tricuspid valve system of the present application also passes through a jugular vein approach, as shown in FIG. 16-17.

The loaded anchoring stent is delivered into the tricuspid valve of the lesion of the right atrium patient from the superior vena cava through the jugular vein, and the following procedure is the same (FIGS. 16-17).

The above embodiments are only intended to fully illustrate the normative embodiments of the present invention. The intervention tricuspid valve system of the present invention has been experimentally implemented in animals and confirmed to be feasible.

The invention can realize the significance that: ① the split design realizes that the valve leaflet support and the valve anchoring are separated in function, the anchoring of the valve to the tricuspid valve position is delivered to the anchoring stent, so that the anchoring personalized design can be realized, and meanwhile, the anchoring stent and the transcatheter valve are inserted step by step, so that can avoid the difficulties in catheter delivery caused by the volume is too large after crimping; ②the anchoring principle and the pre-design and measurement of the final anchoring part are carried out through the anatomical structure characteristics of the lesion valve, and the second anchoring state of the anchoring stent is determined; the size and dimension of each part are constructed through the personalized image data of the patient, the special software and the three-dimensional printing pretest to complete the preset transition state, namely the three-dimensional shaping design and processing of the first anchoring state, so that the catheter is accurately aligned after being released, and the support is provided for smooth delivery of the transcatheter valve. For example, the conical structure of the first state of the anchoring stent can be moderately expanded and narrow, and can also be constrained more severely; the former not only provides a channel for valve intervention, but also can avoid sudden expansion of the stenotic lesion; the former can relieve a large amount of regurgitation of the valve insufficiency, and provides space and time guarantee for the entry of the transcatheter artificial biological tricuspid valve; ③the external force released by the valve is used for driving the anchoring support to be deformed into a cylindrical second state from the funnel-like first anchoring state, and the deformation generates an anchoring stent centripetal gripping valve, so that the zero displacement of the valve is ensured by fitting with the transcatheter valve, and the anchoring hook loop structure is further tightly combined with the lower leaflet tissue by means of the ventricular surface anchoring hook loop structure, so that the predesigned alignment anchoring is completed, and meanwhile, clamping is formed on the anchoring stent and the structure on the valve to achieve final anchoring; ④the arrangement support rod structure of the inflow end and the outflow end of the connecting part of the transcatheter tricuspid valve anchoring stent can be integrated with the transcatheter artificial biological tricuspid valve from both ends to ensure that the valve is not displaced; ⑤in the split type precisely-anchorable transcatheter tricuspid valve system described above, each completion of the transcatheter artificial biological tricuspid valve treatment process accurately anchored for the personalized preset realization, the analysis of related data, the shape design of the transcatheter tricuspid valve anchoring stent, processing and manufacturing, related data obtained in the whole process of interventional treatment and postoperative follow-up visit data and the like, as an independent data unit, a large amount of personalized image data, an anchoring stent design and related data such as processing and manufacturing parameters, a interventional treatment process and a postoperative result are accumulated, and the intelligent, commercialization and large-scale application of the interventional treatment implementation of the split type precisely-anchorable transcatheter tricuspid valve system is gradually realized.

## Claims

1. A split type precisely-anchorable transcatheter tricuspid valve system, comprising a split transcatheter tricuspid valve anchoring stent (10) and a transcatheter artificial biological tricuspid valve (20), **characterized in that**,
the shape and structure of the transcatheter tricuspid valve anchoring stent (10) are matched with a tricuspid valve's real structure after a patient's image data is subjected to three-dimensional reconstruction, the transcatheter tricuspid valve anchoring stent (10) is configured to be firstly delivered to a tricuspid valve site of the patient and is configured to be released and deformed with precise alignment with a supravalvular and subvalvular tissue of the patient's tricuspid valve site;
the transcatheter tricuspid valve anchoring stent (10) has a compressed state disposed in a delivery catheter (31), a first anchoring state after being released by the delivery catheter (31), and a second anchoring state after being combined with the transcatheter artificial biological tricuspid valve (20),
in the first anchoring state, the transcatheter tricuspid valve anchoring stent (10), being processed and shaped into a funnel shape with a large atrial surface (11) and a small ventricular surface (12), deformation and return shape of the transcatheter tricuspid valve anchoring stent (10) after being released through the delivery catheter (31) adapt to the supravalvular and subvalvular tissue of the patient's tricuspid valve site and alignment, engagement and clamping is implemented;
in the first anchoring state, the tricuspid valve anchoring stent (10) is configured to be released by the delivery catheter (31) and then deformed to be in precise alignment, engagement and clamping with the supravalvular and subvalvular tissue of the patient's tricuspid valve site;
in the second anchoring state, the transcatheter artificial biological tricuspid valve (20) is configured to be delivered into the transcatheter tricuspid valve anchoring stent (10) in the first state by the delivery catheter (31) to achieve secondary deformation through expansion by balloon expandable external force, and it is integrated with the expanded transcatheter artificial biological tricuspid valve (20); during the secondary deformation, the transcatheter tricuspid valve anchoring stent (10) deforms from an original funnel shape into a cylindrical shape to generate a centripetal return clip, and a tight combination with the tricuspid valve site and subvalvular tissue of the patient is completed.

2. The split type precisely-anchorable transcatheter tricuspid valve system according to claim 1, **characterized in that**, the tricuspid valve system further comprises a delivery assembly (30), the delivery assembly comprises a transcatheter tricuspid valve anchoring stent delivery set and a transcatheter artificial biological tricuspid valve delivery set, and the transcatheter tricuspid valve anchoring stent delivery set comprises a delivery catheter (31), a transcatheter tricuspid valve anchoring stent delivery device and a stent loader (32).

3. The split type precisely-anchorable transcatheter tricuspid valve system according to claim 1, **characterized in that**, the split type transcatheter tricuspid valve anchoring stent (10) and the transcatheter artificial biological tricuspid valve (20) are configured to be respectively in the anterior and posterior approach, and then reassembled into one in vivo, and meanwhile, the transcatheter tricuspid valve anchoring stent (10) is deformed again to be combined with the tricuspid valve and the subvalvular tissue of the patient's disease to achieve the anchoring of the transcatheter artificial biological tricuspid valve (20), so as to replace the tricuspid valve of the disease in situ.

4. The split type precisely-anchorable transcatheter tricuspid valve system according to claim 1, **characterized in that**,
the transcatheter tricuspid valve anchoring stent (10) is an umbrella tubular stent structure, comprising the large atrial surface (11), the small ventricular surface (12) and an anchoring stent connecting part (13) therebetween; the atrial surface (11) is an umbrella sheet, has an umbrella shape matching a real shape of the three-dimensional reconstruction of the image data of the atrial surface (11) of the patient, and is a first lattice portion; the ventricular surface (12) has three positioning hook loops (121) which are preset with the boundary positions of an anterior leaflet, the septum and a posterior leaflet of the tricuspid valve of the patient; the anchoring stent connecting part (13) is a circular funnel shape forming a second lattice portion having a circular opening diameter matching with an inner diameter of the tricuspid valve rings of the patient and an outer diameter of a subsequent transcatheter artificial biological tricuspid valve, a lower opening of the funnel shape matches with the shape and degree of tricuspid valve stenosis and /or regurgitation diseases.

5. The split type precisely-anchorable transcatheter tricuspid valve system according to claim 4, **characterized in that**, the first anchoring state of the transcatheter tricuspid valve anchoring stent (10) is a three-dimensional shape-memory state of the real anatomical shape and structure of the patient after delivery and release of the delivery catheter (31), the shape-memory state of the anchoring stent connecting part (13) from the atrial surface (11) to the ventricular surface (12) has a contraction taper, the taper is 5-45 degrees, the anchoring stent connecting part (13) is configured to be expanded to deform from the funnel shape in the first anchoring state into the cylindrical shape in the second anchoring state, depending on the shape of a diseased leaflet of the patient.

6. The split type precisely-anchorable transcatheter tricuspid valve system according to claim 5, **characterized in that**, in the first anchoring state of the transcatheter tricuspid valve anchoring stent (10), after the positioning hook loops (121) are released through the delivery catheter (31), the positioning hook loops (121) are inserted into the junction position of the anterior leaflet, the septum and the posterior leaflet of the tricuspid valve of the patient's disease matched with the positioning hook loops (121), so as to realize corresponding placement of the atrial surface (11) and the right atrium of the patient.

7. The split type precisely-anchorable transcatheter tricuspid valve system according to claim 4, **characterized in that**, the ventricular surface (12) has a plurality of anchoring hook loops (122), and the anchoring hook loops (122) are configured to extend from the anchoring stent connecting part (13) to the right ventricular surface (12) and then be folded, so as to accurately match the number of morphological contours of the real chordae tendineae and subvalvular tissue structure shape of the three-dimensional reconstruction of the image data.

8. The split type precisely-anchorable transcatheter tricuspid valve system according to claim 4, **characterized in that**, the atrial surface end portion of the connecting part of the transcatheter tricuspid valve anchoring stent is provided with a plurality of fixed support rods (111) for embedding the stent of the transcatheter artificial biological triscuspid valve (20), and the fixed support rods (111) are bent along the axial direction of the atrial surface (11) and the ends thereof bend toward the axis of the transcatheter tricuspid valve anchoring stent (10).

9. The split type precisely-anchorable transcatheter tricuspid valve system according to claim 8, **characterized in that**, in the first anchoring state of the transcatheter tricuspid valve anchoring stent (10), the fixed support rods (111) maintain an angle consistent with the anchoring stent connecting part (13), and in the second anchoring state of the transcatheter tricuspid valve anchoring stent (10), the plurality of fixed support rods (11) are axially parallel to the coaptation circumference, so that the ends of the fixed support rods (111) are embedded on the stent of the inflow end of the transcatheter artificial biological tricuspid valve (20), and structurally integrate with the transcatheter artificial biological tricuspid valve (20) to realize zero displacement of the release of the transcatheter artificial biological tricuspid valve (20).

10. The split type precisely-anchorable transcatheter tricuspid valve system according to claim 8, **characterized in that**, there are 3-12 fixed support rods (111).

11. The split type precisely-anchorable transcatheter tricuspid valve system according to claim 1, **characterized in that**, a surface of the transcatheter tricuspid valve anchoring stent (10) is partially or completely coated with a layer of medical polymer film.

12. The split type precisely-anchorable transcatheter tricuspid valve system according to claim 4, **characterized in that**, the atrial surface (11), the ventricular surface (12) and the anchoring stent connecting part (13) are three-dimensional shaped structures formed by laser cutting; or they are reconnected after a split processing.

13. The split type precisely-anchorable transcatheter tricuspid valve system according to claim 1, **characterized in that**, the transcatheter tricuspid valve anchoring stent (10) is a metallic material or a non-metallic material having shape-memory reshape properties.

14. The split type precisely-anchorable transcatheter tricuspid valve system according to claim 1, **characterized in that**, the transcatheter tricuspid valve anchoring stent (10) is made of a nickel-titanium alloy material.

15. The split type precisely-anchorable transcatheter tricuspid valve system according to claim 1, **characterized in that**, the transcatheter artificial biological tricuspid valve (20) comprises a radially compressible cobalt-chromium alloy stent which can be expanded after balloon expansion, and three fan-shaped leaflets arranged on the inner side of the stent, any of the fan-shaped leaflets has a free edge, an arc-shaped bottom edge and leaflet boundary connecting parts extending on the two sides, and the stent is a metal net tube or a valve stent which can support three commissures of three leaflet interfaces and can be crimped.

16. The split type precisely-anchorable transcatheter tricuspid valve system according to claim 15, **characterized in that**, the valve stent is a cobalt-based alloy cobalt or chromium alloy or a nickel-titanium alloy.

17. The split type precisely-anchorable transcatheter tricuspid valve system according to claim 2, **characterized in that**, the transcatheter artificial biological tricuspid valve delivery set comprises a transcatheter artificial biological tricuspid valve delivery device, a guide sheath, a valve holder, and a charging pump.

18. The split type precisely-anchorable transcatheter tricuspid valve system according to claim 17, **characterized in that**, the transcatheter tricuspid valve anchoring stent delivery device and the transcatheter artificial tricuspid valve delivery device can be delivered from the inferior vena cava via the femoral vein, or from the superior vena cava via the jugular vein or subclavian vein to the tricuspid valve site.

19. The split type precisely-anchorable transcatheter tricuspid valve system according to claim 4, **characterized in that**, the transcatheter tricuspid valve anchoring stent (10) is configured to be firstly guided into a diseased tricuspid valve site of the patient through the delivery catheter (31) and released into the first anchoring state, and the transcatheter artificial biological tricuspid valve (20) is configured to be sent into the anchoring stent through the delivery catheter (31), and as the transcatheter artificial biological tricuspid valve (20) is expanded, the transcatheter tricuspid valve anchoring stent (10) is configured to be expanded to the second anchoring state, and the anchoring stent connecting part (13) and the transcatheter artificial biological tricuspid valve (20) are fitted and the ventricular surface (12) of the stent completes further tight combination with the subvalvular structure to form final anchoring.

## Patentansprüche

1. Präzise verankerbares Transkathetertrikuspidalklappensystem einer geteilten Art, umfassend einen geteilten Transkathetertrikuspidalklappenverankerungsstent (10) und eine künstliche biologische Transkathetertrikuspidalklappe (20), **dadurch gekennzeichnet, dass**
die Form und Struktur des Transkathetertrikuspidalklappenverankerungsstents (10) mit einer echten Struktur einer Trikuspidalklappe in Übereinstimmung gebracht sind, nachdem Bilddaten eines Patienten einer dreidimensionalen Rekonstruktion unterzogen wurden, wobei der Transkathetertrikuspidalklappenverankerungsstent (10) konfiguriert ist, um zuerst an eine Trikuspidalklappenstelle des Patienten zugeführt zu werden, und konfiguriert ist, um mit präziser Ausrichtung auf ein supravalvuläres und subvalvuläres Gewebe der Trikuspidalklappenstelle des Patienten freigegeben und verformt zu werden;
der Transkathetertrikuspidalklappenverankerungsstent (10) einen in einem Zuführkatheter (31) angeordneten komprimierten Zustand einen ersten Verankerungszustand aufweist nachdem er mittels des Zuführkatheters (31) freigegeben wird, und einen zweiten Verankerungszustand aufweist, nachdem er mit der künstlichen biologischen Transkathetertrikuspidalklappe (20) kombiniert wird,
in dem ersten Verankerungszustand, der Transkathetertrikuspidalklappenverankerungsstent (10) zu einer Trichterform mit einer grossen atrialen Oberfläche (11) und einer kleinen ventrikulären Oberfläche (12) verarbeitet und geformt ist, sich eine Verformung und Rückstellform des Transkathetertrikuspidalklappenverankerungsstents (10), nachdem er durch den Zuführkatheter (31) freigegeben wird, an das supravalvuläre und subvalvuläre Gewebe der Trikuspidalklappenstelle des Patienten anpassen und eine Ausrichtung, ein Eingriff und eine Klemmung implementiert werden;
in dem ersten Verankerungszustand, der Trikuspidalklappenverankerungsstent (10) konfiguriert ist, um mittels des Zuführkatheters (31) freigegeben und dann verformt zu werden, um in präziser Ausrichtung, in Eingriff und in Klemmung mit dem supravalvulären und subvalvulären Gewebe der Trikuspidalklappenstelle des Patienten zu sein;
in dem zweiten Verankerungszustand, die künstliche biologische Transkathetertrikuspidalklappe (20) konfiguriert ist, um in dem ersten Zustand mittels des Zuführkatheters (31) in den Transkathetertrikuspidalklappenverankerungsstent (10) zugeführt zu werden, um eine sekundäre Verformung durch Expansion mittels einer ballonexpandierbaren äusseren Kraft zu erreichen, und sie in die expandierte künstliche biologische Transkathetertrikuspidalklappe (20) integriert ist; während der sekundären Verformung, sich der Transkathetertrikuspidalklappenverankerungsstent (10) von einer ursprünglichen Trichterform in eine zylindrische Form verformt, um einen zentripetalen Rückstellclip zu erzeugen, und eine enge Kombination mit der Trikuspidalklappenstelle und dem subvalvulären Gewebe des Patienten abgeschlossen wird.

2. Präzise verankerbares Transkathetertrikuspidalklappensystem der geteilten Art nach Anspruch 1, **dadurch gekennzeichnet, dass** das Trikuspidalklappensystem ferner eine Zuführbaugruppe (30) umfasst, die Zuführbaugruppe einen Zuführsatz für den Transkathetertrikuspidalklappenverankerungsstent und einen Zuführsatz für die künstliche biologische Trikuspidalklappe umfasst, und der Zuführsatz für den Transkathetertrikuspidalklappenverankerungsstent einen Zuführkatheter (31), eine Zuführvorrichtung für den Transkathetertrikuspidalklappenverankerungsstent und einen Stentlader (32) umfasst.

3. Präzise verankerbares Transkathetertrikuspidalklappensystem der geteilten Art nach Anspruch 1, **dadurch gekennzeichnet, dass** der geteilte Transkathetertrikuspidalklappenverankerungsstent (10) und die künstliche biologische Transkathetertrikuspidalklappe (20) konfiguriert sind, um sich jeweils in dem anterioren und posterioren Zugang zu befinden und dann in vivo zu einem wieder zusammengesetzt werden, und währenddessen der Transkathetertrikuspidalklappenverankerungsstent (10) erneut verformt wird, um mit der Trikuspidalklappe und dem subvalvulären Gewebe einer Erkrankung des Patienten kombiniert zu werden, um die Verankerung der künstlichen biologischen Transkathetertrikuspidalklappe (20) zu erreichen, um die Trikuspidalklappe der Erkrankung in situ zu ersetzen.

4. Präzise verankerbares Transkathetertrikuspidalklappensystem der geteilten Art nach Anspruch 1, **dadurch gekennzeichnet, dass**,
der Transkathetertrikuspidalklappenverankerungsstent (10) eine röhrenförmige Schirmstentstruktur ist, umfassend die grosse atriale Oberfläche (11), die kleine ventrikuläre Oberfläche (12) und ein Verankerungsstentverbindungsteil (13) dazwischen; die atriale Oberfläche (11) ein Schirmblatt ist, eine Schirmform aufweist, die mit einer echten Form der dreidimensionalen Rekonstruktion der Bilddaten der atrialen Oberfläche (11) des Patienten übereinstimmt, und ein erster Gitterabschnitt ist; die ventrikuläre Oberfläche (12) drei Positionierungshakenschlaufen (121) aufweist, die mit den Begrenzungspositionen eines anterioren Segels, des Septums und eines posterioren Segels der Trikuspidalklappe des Patienten voreingestellt sind; das Verankerungsstentverbindungsteil (13) eine kreisförmige Trichterform ist, die einen zweiten Gitterabschnitt ausbildet, der einen Durchmesser einer kreisförmigen Öffnung aufweist, der mit einem Innendurchmesser der Trikuspidalklappenringe des Patienten und einem Aussendurchmesser einer nachfolgenden künstlichen biologischen Transkathetertrikuspidalklappe übereinstimmt, wobei eine untere Öffnung der Trichterform mit der Form und dem Grad einer Stenose und/oder Insuffizienzerkrankungen der Trikuspidalklappe übereinstimmt.

5. Präzise verankerbares Transkathetertrikuspidalklappensystem der geteilten Art nach Anspruch 4, **dadurch gekennzeichnet, dass** der erste Verankerungszustand des Transkathetertrikuspidalklappenverankerungsstents (10) ein dreidimensionaler Formgedächtniszustand der echten anatomischen Form und Struktur des Patienten nach einer Zuführung und Freigabe des Zuführkatheters (31) ist, der Formgedächtniszustand des Verankerungsstentverbindungsteils (13) von der atrialen Oberfläche (11) zu der ventrikulären Oberfläche (12) eine Kontraktionsverjüngung aufweist, die Verjüngung 5-45 Grad beträgt, das Verankerungsstentverbindungsteil (13) konfiguriert ist, um von der Trichterform im ersten Verankerungszustand in die zylindrische Form im zweiten Verankerungszustand expandiert zu werden, um sich in Abhängigkeit von der Form eines erkrankten Segels des Patienten zu verformen.

6. Präzise verankerbares Transkathetertrikuspidalklappensystem der geteilten Art nach Anspruch 5, **dadurch gekennzeichnet, dass**, in dem ersten Verankerungszustand des Transkathetertrikuspidalklappenverankerungsstents (10), nachdem die Positionierungshakenschlaufen (121) durch den Zuführkatheter (31) freigegeben wurden, die Positionierungshakenschlaufen (121) in die Knotenposition des vorderen Segels, des Septums und des hinteren Segels der Trikuspidalklappe der Erkrankung des Patienten eingeführt werden, die mit den Positionierungshakenschlaufen (121) in Übereinstimmung gebracht ist, um eine entsprechende Platzierung der atrialen Oberfläche (11) und des rechten Vorhofs des Patienten zu realisieren.

7. Präzise verankerbares Transkathetertrikuspidalklappensystem der geteilten Art nach Anspruch 4, **dadurch gekennzeichnet, dass** die ventrikuläre Oberfläche (12) eine Vielzahl von Verankerungshakenschlaufen (122) aufweist, und die Verankerungshakenschlaufen (122) konfiguriert sind, um sich von dem Verankerungsstentverbindungsteil (13) zu der rechten ventrikulären Oberfläche (12) zu erstrecken und dann gefaltet zu werden, um mit der Anzahl von morphologischen Konturen der echten Chordae tendineae und der subvalvulären Gewebestrukturform der dreidimensionalen Rekonstruktion der Bilddaten genau übereinzustimmen.

8. Präzise verankerbares Transkathetertrikuspidalklappensystem der geteilten Art nach Anspruch 4, **dadurch gekennzeichnet, dass** der Endabschnitt der atrialen Oberfläche des Verbindungsteils des Transkathetertrikuspidalklappenverankerungsstents mit einer Vielzahl von festen Stützstäben (111) zum Einbetten des Stents der künstlichen biologischen Transkathetertrikuspidalklappe (20) bereitgestellt ist,
und die festen Stützstäbe (111) entlang der axialen Richtung der atrialen Oberfläche (11) gebogen sind und sich die Enden davon in Richtung der Achse des Transkathetertrikuspidalklappenverankerungsstents (10) biegen.

9. Präzise verankerbares Transkathetertrikuspidalklappensystem der geteilten Art nach Anspruch 8, **dadurch gekennzeichnet, dass**, in dem ersten Verankerungszustand des Transkathetertrikuspidalklappenverankerungsstents (10), die festen Stützstäbe (111) einen Winkel im Einklang mit dem Verankerungsstentverbindungsteil (13) beibehalten und, in dem zweiten Verankerungszustand des Transkathetertrikuspidalklappenverankerungsstents (10), die Vielzahl von festen Stützstäben (11) axial parallel zu dem Koaptationsumfang sind, sodass die Enden der festen Stützstäbe (111) an dem Stent des Einströmendes der künstlichen biologischen Transkathetertrikuspidalklappe (20) eingebettet sind und sich in die künstliche biologische Transkathetertrikuspidalklappe (20) strukturell integrieren, um eine Nullverschiebung der Freigabe der künstlichen biologischen Transkathetertrikuspidalklappe (20) zu realisieren.

10. Präzise verankerbares Transkathetertrikuspidalklappensystem der geteilten Art nach Anspruch 8, **dadurch gekennzeichnet, dass** 3-12 feste Stützstäbe (111) vorhanden sind.

11. Präzise verankerbares Transkathetertrikuspidalklappensystem der geteilten Art nach Anspruch 1, **dadurch gekennzeichnet, dass** eine Oberfläche des Transkathetertrikuspidalklappenverankerungsstents (10) mit einer Schicht aus medizinischer Polymerfolie teilweise oder vollständig beschichtet ist.

12. Präzise verankerbares Transkathetertrikuspidalklappensystem der geteilten Art nach Anspruch 4, **dadurch gekennzeichnet, dass** die atriale Oberfläche (11), die ventrikuläre Oberfläche (12) und das Verankerungsstentverbindungsteil (13) mittels eines Laserschneidens ausgebildete, dreidimensional geformte Strukturen sind; oder sie nach einer geteilten Verarbeitung wieder verbunden werden.

13. Präzise verankerbares Transkathetertrikuspidalklappensystem der geteilten Art nach Anspruch 1, **dadurch gekennzeichnet, dass** der Transkathetertrikuspidalklappenverankerungsstent (10) ein metallisches Material oder ein nichtmetallisches Material ist, das Formgedächtnisumformeigenschaften aufweist.

14. Präzise verankerbares Transkathetertrikuspidalklappensystem der geteilten Art nach Anspruch 1, **dadurch gekennzeichnet, dass** der Transkathetertrikuspidalklappenverankerungsstent (10) aus einem Nickel-Titan-Legierungsmaterial hergestellt ist.

15. Präzise verankerbares Transkathetertrikuspidalklappensystem der geteilten Art nach Anspruch 1, **dadurch gekennzeichnet, dass** die künstliche biologische Transkathetertrikuspidalklappe (20) einen radial komprimierbaren Stent mit Kobalt-Chrom-Legierung, der nach einer Ballonexpansion expandiert werden kann, und drei fächerförmige, an der Innenseite des Stents angeordnete Segel aufweist, wobei jedes der fächerförmigen Segel einen freien Rand, einen bogenförmigen unteren Rand und sich auf den zwei Seiten erstreckende Segelbegrenzungsverbindungsteile aufweist, und der Stent ein Metallnetzrohr oder ein Klappenstent ist, der drei Kommissuren von drei Segelgrenzflächen stützen kann und gecrimpt werden kann.

16. Präzise verankerbares Transkathetertrikuspidalklappensystem der geteilten Art nach Anspruch 15, **dadurch gekennzeichnet, dass** der Klappenstent eine kobaltbasierte Legierung, eine Kobalt- oder Chromlegierung oder eine Nickel-Titan-Legierung ist.

17. Präzise verankerbares Transkathetertrikuspidalklappensystem der geteilten Art nach Anspruch 2, **dadurch gekennzeichnet, dass** der Zuführsatz für die künstliche biologische Transkathetertrikuspidalklappe eine Zuführvorrichtung für die künstliche biologische Transkathetertrikuspidalklappe, eine Führungshülse, einen Klappenhalter und eine Ladepumpe umfasst.

18. Präzise verankerbares Transkathetertrikuspidalklappensystem der geteilten Art nach Anspruch 17, **dadurch gekennzeichnet, dass** die Zuführvorrichtung für den Transkathetertrikuspidalklappenverankerungsstent und die Zuführvorrichtung für die künstliche Transkathetertrikuspidalklappe von der Vena cava inferior über die Oberschenkelvene oder von der Vena cava superior über die Halsvene oder Schlüsselbeinvene an die Trikuspidalklappenstelle zugeführt werden können.

19. Präzise verankerbares Transkathetertrikuspidalklappensystem der geteilten Art nach Anspruch 4, **dadurch gekennzeichnet, dass** der Transkathetertrikuspidalklappenverankerungsstent (10) konfiguriert ist, um zuerst durch den Zuführkatheter (31) in eine erkrankte Trikuspidalklappenstelle des Patienten geführt und in den ersten Verankerungszustand freigegeben zu werden, und die künstliche biologische Transkathetertrikuspidalklappe (20) konfiguriert ist, um durch den Zuführkatheter (31) in den Verankerungsstent befördert zu werden, und wenn die künstliche biologische Transkathetertrikuspidalklappe (20) expandiert wird, der Transkathetertrikuspidalklappenverankerungsstent (10) konfiguriert ist, um in den zweiten Verankerungszustand expandiert zu werden, und das Verankerungsstentverbindungsteil (13) und die künstliche biologische Transkathetertrikuspidalklappe (20) eingepasst werden und die ventrikuläre Oberfläche (12) des Stents eine weitere enge Kombination mit der subvalvulären Struktur abschliesst, um eine endgültige Verankerung auszubilden.

## Revendications

1. Système de valve tricuspide transcathéter à ancrage précis de type divisé, comprenant un stent d'ancrage de valve tricuspide transcathéter divisé (10) et une valve tricuspide biologique artificielle transcathéter (20), **caractérisé en ce que**,
la forme et la structure du stent d'ancrage de valve tricuspide transcathéter (10) sont mises en correspondance avec la structure réelle d'une valve tricuspide après que les données d'imagerie d'un patient ont été soumises à une reconstruction tridimensionnelle, le stent d'ancrage de valve tricuspide transcathéter (10) est conçu pour être d'abord acheminé vers un site de valve tricuspide du patient et est conçu pour être libéré et déformé avec un alignement précis avec un tissu supravalvulaire et sous-valvulaire du site de valve tricuspide du patient;
le stent d'ancrage de valve tricuspide transcathéter (10) a un état comprimé disposé dans un cathéter d'acheminement (31), un premier état d'ancrage après avoir été libéré par le cathéter d'acheminement (31), et un second état d'ancrage après avoir été combiné avec la valve tricuspide biologique artificielle transcathéter (20),
dans le premier état d'ancrage, le stent d'ancrage de valve tricuspide transcathéter (10) étant traité et conformé en forme d'entonnoir avec une grande surface atriale (11) et une petite surface ventriculaire (12), la déformation et la forme de retour du stent d'ancrage de valve tricuspide transcathéter (10) après avoir été libéré à travers le cathéter d'acheminement (31) s'adaptent au tissu supravalvulaire et sous-valvulaire du site de valve tricuspide du patient et l'alignement, la mise en prise et le serrage sont mis en œuvre;
dans le premier état d'ancrage, le stent d'ancrage de valve tricuspide (10) est conçu pour être libéré par le cathéter d'acheminement (31) puis déformé pour être en alignement, mise en prise et serrage précis avec le tissu supravalvulaire et sous-valvulaire du site de valve tricuspide du patient;
dans le second état d'ancrage, la valve tricuspide biologique artificielle transcathéter (20) est conçue pour être acheminée dans le stent d'ancrage de valve tricuspide transcathéter (10) dans le premier état par le cathéter d'acheminement (31) pour obtenir une déformation secondaire par expansion au moyen d'une force externe expansible par ballonnet, et elle est intégrée à la valve tricuspide biologique artificielle transcathéter (20) expansée; lors de la déformation secondaire, le stent d'ancrage de valve tricuspide transcathéter (10) se déforme d'une forme d'origine en entonnoir à une forme cylindrique pour générer une pince de retour centripète, et une combinaison étroite avec le site de valve tricuspide et le tissu sous-valvulaire du patient est effectuée.

2. Système de valve tricuspide transcathéter à ancrage précis de type divisé selon la revendication 1, **caractérisé en ce que**, le système de valve tricuspide comprend en outre un ensemble d'acheminement (30), l'ensemble d'acheminement comprend un jeu d'acheminement de stent d'ancrage de valve tricuspide transcathéter et un jeu d'acheminement de valve tricuspide biologique artificielle transcathéter, et le jeu d'acheminement de stent d'ancrage de valve tricuspide transcathéter comprend un cathéter d'acheminement (31), un dispositif d'acheminement de stent d'ancrage de valve tricuspide transcathéter et un chargeur de stent (32).

3. Système de valve tricuspide transcathéter à ancrage précis de type divisé selon la revendication 1, **caractérisé en ce que**, le stent d'ancrage de valve tricuspide transcathéter de type divisé (10) et la valve tricuspide biologique artificielle transcathéter (20) sont conçus pour être respectivement dans la voie d'abord antérieure et la voie d'abord postérieure, puis réassemblés en un seul bloc in vivo, et pendant ce temps, le stent d'ancrage de valve tricuspide transcathéter (10) est à nouveau déformé pour être combiné avec la valve tricuspide et le tissu sous-valvulaire de la maladie du patient pour effectuer l'ancrage de la valve tricuspide biologique artificielle transcathéter (20), de sorte à remplacer la valve tricuspide pathologique in situ.

4. Système de valve tricuspide transcathéter à ancrage précis de type divisé selon la revendication 1, **caractérisé en ce que**,
le stent d'ancrage de valve tricuspide transcathéter (10) est une structure de stent tubulaire en parapluie, comprenant la grande surface atriale (11), la petite surface ventriculaire (12) et une partie de liaison de stent d'ancrage (13) située entre celles-ci; la surface atriale (11) est une feuille en parapluie, a une forme de parapluie correspondant à une forme réelle de la reconstruction tridimensionnelle des données d'imagerie de la surface atriale (11) du patient, et est une première portion de treillis; la surface ventriculaire (12) a trois boucles de crochet de positionnement (121) qui sont préétablies avec les positions limites d'un feuillet antérieur, du septum et d'un feuillet postérieur de la valve tricuspide du patient; la partie de liaison de stent d'ancrage (13) est en forme d'entonnoir circulaire formant une seconde portion de treillis ayant un diamètre d'ouverture circulaire correspondant à un diamètre interne des anneaux de valve tricuspide du patient et à un diamètre externe d'une valve tricuspide biologique artificielle transcathéter ultérieure, une ouverture inférieure de la forme d'entonnoir correspond à la forme et au degré de sténose de valve tricuspide et/ou de maladies de régurgitation.

5. Système de valve tricuspide transcathéter à ancrage précis de type divisé selon la revendication 4, **caractérisé en ce que**, le premier état d'ancrage du stent d'ancrage de valve tricuspide transcathéter (10) est un état à mémoire de forme tridimensionnelle de la forme et de la structure anatomiques réelles du patient après l'acheminement et la libération du cathéter d'acheminement (31), l'état à mémoire de forme de la partie de liaison de stent d'ancrage (13) de la surface atriale (11) à la surface ventriculaire (12) a une conicité de contraction, la conicité est de 5 à 45 degrés, la partie de liaison de stent d'ancrage (13) est conçue pour être expansée pour se déformer de la forme en entonnoir dans le premier état d'ancrage à la forme cylindrique dans le second état d'ancrage, en fonction de la forme d'un feuillet pathologique du patient.

6. Système de valve tricuspide transcathéter à ancrage précis de type divisé selon la revendication 5, **caractérisé en ce que**, dans le premier état d'ancrage du stent d'ancrage de valve tricuspide transcathéter (10), après que les boucles de crochet de positionnement (121) ont été libérées à travers le cathéter d'acheminement (31), les boucles de crochet de positionnement (121) sont insérées dans la position de jonction du feuillet antérieur, du septum et du feuillet postérieur de la valve tricuspide de la maladie du patient correspondant aux boucles de crochet de positionnement (121), de sorte à réaliser un placement correspondant de la surface atriale (11) et de l'atrium droit du patient.

7. Système de valve tricuspide transcathéter à ancrage précis de type divisé selon la revendication 4, **caractérisé en ce que**, la surface ventriculaire (12) a une pluralité de boucles de crochet d'ancrage (122), et les boucles de crochet d'ancrage (122) sont conçues pour s'étendre de la partie de liaison de stent d'ancrage (13) à la surface ventriculaire droite (12) puis être pliées, de sorte à correspondre avec exactitude au nombre de contours morphologiques des cordages tendineux réels et à la forme de la structure du tissu sous-valvulaire de la reconstruction tridimensionnelle des données d'imagerie.

8. Système de valve tricuspide transcathéter à ancrage précis de type divisé selon la revendication 4, **caractérisé en ce que**, la portion d'extrémité de surface atriale de la partie de liaison du stent d'ancrage de valve tricuspide transcathéter est pourvue d'une pluralité de tiges de support fixes (111) permettant d'encastrer le stent de la valve tricuspide biologique artificielle transcathéter (20),
et les tiges de support fixes (111) sont courbées le long de la direction axiale de la surface atriale (11) et les extrémités de celles-ci se courbent vers l'axe du stent d'ancrage de valve tricuspide transcathéter (10).

9. Système de valve tricuspide transcathéter à ancrage précis de type divisé selon la revendication 8, **caractérisé en ce que**, dans le premier état d'ancrage du stent d'ancrage de valve tricuspide transcathéter (10), les tiges de support fixes (111) maintiennent un angle cohérent avec la partie de liaison de stent d'ancrage (13), et dans le second état d'ancrage du stent d'ancrage de valve tricuspide transcathéter (10), la pluralité de tiges de support fixes (11) sont axialement parallèles à la circonférence de coaptation, de sorte que les extrémités des tiges de support fixes (111) sont encastrées sur le stent de l'extrémité d'entrée de la valve tricuspide biologique artificielle transcathéter (20), et s'intègrent structurellement à la valve tricuspide biologique artificielle transcathéter (20) pour réaliser un déplacement nul de la libération de la valve tricuspide biologique artificielle transcathéter (20).

10. Système de valve tricuspide transcathéter à ancrage précis de type divisé selon la revendication 8, **caractérisé en ce qu'**il y a 3 à 12 tiges de support fixes (111).

11. Système de valve tricuspide transcathéter à ancrage précis de type divisé selon la revendication 1, **caractérisé en ce qu'**une surface du stent d'ancrage de valve tricuspide transcathéter (10) est partiellement ou complètement revêtue d'une couche de film polymère médical.

12. Système de valve tricuspide transcathéter à ancrage précis de type divisé selon la revendication 4, **caractérisé en ce que**, la surface atriale (11), la surface ventriculaire (12) et la partie de liaison de stent d'ancrage (13) sont des structures de forme tridimensionnelle façonnées par découpe au laser; ou ils sont reliés à nouveau après un traitement divisé.

13. Système de valve tricuspide transcathéter à ancrage précis de type divisé selon la revendication 1, **caractérisé en ce que**, le stent d'ancrage de valve tricuspide transcathéter (10) est un matériau métallique ou un matériau non métallique ayant des propriétés de remise en forme à mémoire de forme.

14. Système de valve tricuspide transcathéter à ancrage précis de type divisé selon la revendication 1, **caractérisé en ce que**, le stent d'ancrage de valve tricuspide transcathéter (10) est constitué d'un matériau d'alliage de nickel-titane.

15. Système de valve tricuspide transcathéter à ancrage précis de type divisé selon la revendication 1, **caractérisé en ce que**, la valve tricuspide biologique artificielle transcathéter (20) comprend un stent en alliage de cobalt-chrome radialement compressible qui peut être expansée après une expansion par ballonnet, et trois feuillets en forme d'éventail agencés sur le côté interne du stent, l'un quelconque des feuillets en forme d'éventail a un bord libre, un bord inférieur en forme d'arc et des parties de liaison de limite de feuillet s'étendant sur les deux côtés, et le stent est un tube maillé métallique ou un stent de valve qui peut supporter trois commissures de trois interfaces de feuillet et peut être sertie.

16. Système de valve tricuspide transcathéter à ancrage précis de type divisé selon la revendication 15, **caractérisé en ce que**, le stent de valve est un alliage à base de cobalt, un alliage de cobalt ou de chrome ou un alliage de nickel-titane.

17. Système de valve tricuspide transcathéter à ancrage précis de type divisé selon la revendication 2, **caractérisé en ce que**, le jeu d'acheminement de valve tricuspide biologique artificielle transcathéter comprend un dispositif d'acheminement de valve tricuspide biologique artificielle transcathéter, une gaine de guidage, un support de valve, et une pompe de chargement.

18. Système de valve tricuspide transcathéter à ancrage précis de type divisé selon la revendication 17, **caractérisé en ce que**, le dispositif d'acheminement de stent d'ancrage de valve tricuspide transcathéter et le dispositif d'acheminement de valve tricuspide artificielle transcathéter peuvent être acheminés de la veine cave inférieure par l'intermédiaire de la veine fémorale, ou de la veine cave supérieure par l'intermédiaire de la veine jugulaire ou de la veine subclavière au site de valve tricuspide.

19. Système de valve tricuspide transcathéter à ancrage précis de type divisé selon la revendication 4, **caractérisé en ce que**, le stent d'ancrage de valve tricuspide transcathéter (10) est conçu pour être d'abord guidé dans un site de valve tricuspide pathologique du patient à travers le cathéter d'acheminement (31) et libérée dans le premier état d'ancrage, et la valve tricuspide biologique artificielle transcathéter (20) est conçue pour être envoyée dans le stent d'ancrage à travers le cathéter d'acheminement (31), et à mesure que la valve tricuspide biologique artificielle transcathéter (20) est expansée, le stent d'ancrage de valve tricuspide transcathéter (10) est conçu pour être expansé dans le second état d'ancrage, et la partie de liaison de stent d'ancrage (13) et la valve tricuspide biologique artificielle transcathéter (20) sont ajustées et la surface ventriculaire (12) du stent effectue une combinaison étroite supplémentaire avec la structure sous-valvulaire pour façonner l'ancrage final.
